# EUROPEAN PATENT APPLICATION

(11) **EP 4 843 390 A1**
(43) Date of publication of application: **25.11.2026**
(21) Application number: 25178644.8
(22) Date of filing: 23.05.2025
(51) Int. Cl.: A01N 63/27, A01P 1/00, A01P 3/00, C12N 1/00

(54) **BACTERIAL STRAINS AND USES THEREOF**

(71) Applicant: PfBIO Ltd, Norwich Norfolk NR4 7UG (GB)
(72) Inventor: CAMPILONGO, Rosaria, Norwich, NR2 3HY (GB); TRUMAN, Andrew William, Wymondham, NR18 0DB (GB); MALONE, Jacob George, Hethersett, NR9 3QH (GB)
(74) Representative: J A Kemp LLP

(57) **Abstract**

Provided herein are *Pseudomonas* bacteria, or extracts thereof for use in treating or preventing eukaryotic and bacterial infections in plants. Also provided herein are *Pseudomonas* strains, or extracts thereof, for use in promoting plant growth.

## Description

### TECHNICAL FIELD

The present disclosure relates to the use of a *Pseudomonas* bacterium, or extracts thereof, in treating or preventing eukaryotic and bacterial infections in plants. The present disclosure also relates to the use of *Pseudomonas* strains, or extracts thereof, in promoting plant growth.

### BACKGROUND

Eukaryotic and bacterial infections in plants cause significant crop losses in both the field and in storage, particularly within the processing sector. While some diseases can be controlled by chemical and synthetic pesticides, these can be damaging to the environment, causing losses in biodiversity and soil depletion. Many pesticides are being withdrawn from the market on environmental grounds, and few products exist to take their place. For a growing number of crop diseases, few, if any, effective pesticides are available. There is therefore an urgent need to develop sustainable methods to control crop pathogens.

With the ever-increasing human population, there is an increasing demand for food. Modern intensive agriculture requires substantial inorganic fertiliser inputs, incurring major, negative impacts on biodiversity and the natural environment. Despite these substantial inputs, yield gains from the application of chemical fertilisers are stagnating, raising concerns about the long-term sustainability of current agricultural practices. New technologies are needed to improve crop yields, either through manipulation of the crops themselves or by sustainably optimising the growth conditions of crops to increase the yield per plant.

### SUMMARY OF THE INVENTION

There is a need to provide alternative treatments to chemical and synthetic pesticides for treating eukaryotic and bacterial infections in plants. There is also a need to increase crop yields. The present invention provides *Pseudomonas* strains, or extracts thereof, suitable for both preventing and treating eukaryotic and bacterial infections in plants as well as stimulating plant growth, thereby increasing crop yields.

The invention provides a method of treating or preventing a eukaryotic and/or bacterial infection in a plant or promoting growth in a plant, the method comprising administering a *Pseudomonas* bacterium, or extract thereof, to the plant.

The invention also provides a method of producing a *Pseudomonas* extract, the method comprising: a) culturing a *Pseudomonas* bacterium under suitable conditions; b) soaking the *Pseudomonas* culture in an extraction solution; c) filtering the *Pseudomonas* culture and extraction solution; d) drying the *Pseudomonas* filtrate; and e) resuspending the *Pseudomonas* filtrate.

The invention further provides an isolated *Pseudomonas* bacterium of PfBIO_Fn1 deposited under accession number 507455 at CABI (i.e. at Centre for Agriculture and Bioscience International, Bakeham Lane Egham, Surrey, TW20 9TY, UK) and/or PfBIO_Fn2 deposited under accession number 507456 at CABI (i.e. at Centre for Agriculture and Bioscience International, Bakeham Lane Egham, Surrey, TW20 9TY, UK).

The invention additionally provides use of a *Pseudomonas* bacterium for treating or preventing a eukaryotic and/or bacterial infection in a plant and/or for promoting growth in a plant.

The invention also provides a composition comprising an isolated *Pseudomonas* bacterium of the invention or a *Pseudomonas* extract obtained by the method of the invention.

### BRIEF DESCRIPTION OF THE DRAWINGS

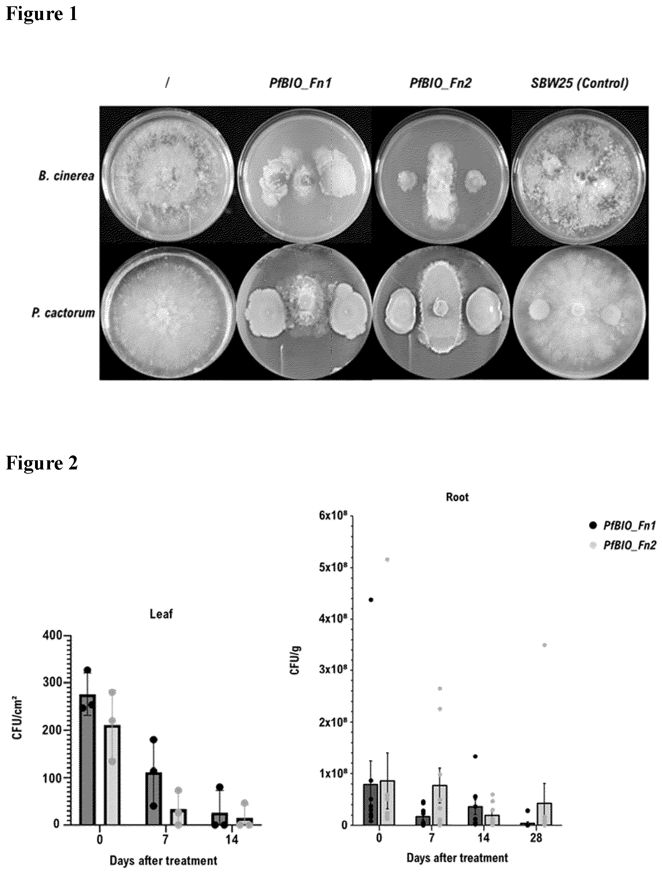

**Figure 1** shows inhibition assays performed on Potato Dextrose Agar (PDA) and V8 medium plates, incubated at 28°C for 7 days, to test the activity of the biocontrol strains, *PfBIO_Fn1* and *PfBIO_Fn2* respectively against *B. cinerea* and *P. cactorum.* Both *PfBIO_Fn1* and *PfBIO_Fn2* show strong activity against the tested pathogens compared to the control strain, *Pseudomonas fluorescens SBW25.*
**Figure 2** shows bacterial survival assays performed on strawberry leaves and roots in soilless media (coir), incubated in a controlled environment room (temperature 20°C during day time, and 15°C at night, Humidity 70%) for 14 days. Samples of leaves were taken and processed after 7 and 14 days. Samples of roots were taken and processed after 7, 14, and 28 days. *PfBIO_Fn1* and *PfBIO_Fn2* survive on both leaves and roots at least until 14 days and 28 days respectively.

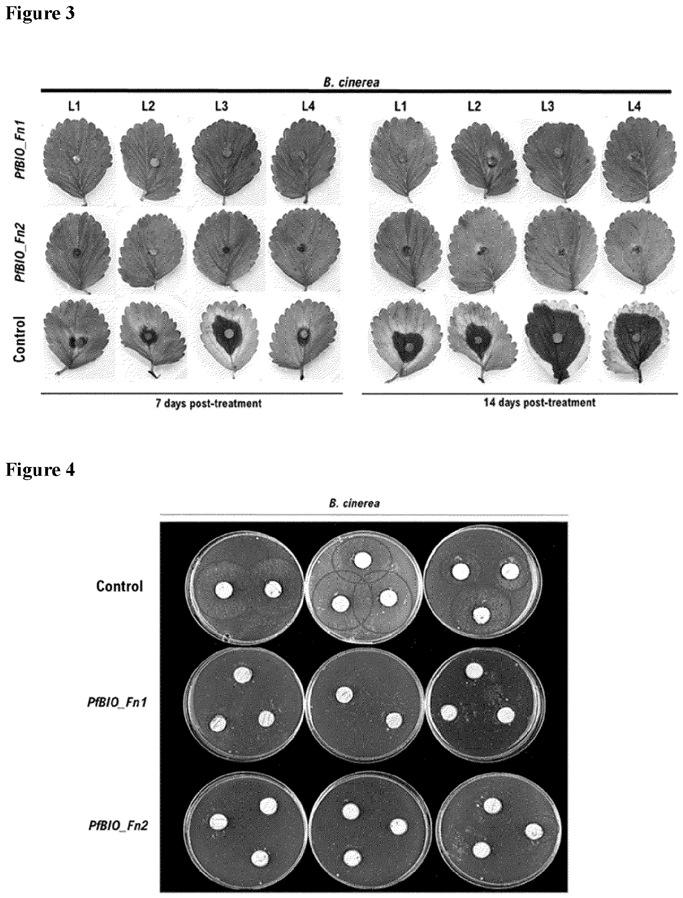

**Figure 3** shows strawberry leaves divided into three groups of four leaves: *PfBIO_Fn1, PfBIO_Fn2,* and Control. The *PfBIO_Fn1* and *PfBIO_Fn2* groups were treated respectively with the strains *PfBIO_Fn1* and *PfBI_Fn2* (both individually resuspended in 10 mM MgCl₂), while the Control group was treated with 10 mM MgCl₂ only. A PDA plug containing *B. cinerea* was placed on top of the leaves that were then incubated at room temperature for two weeks. Both strains *PfBIO_Fn1* and *PfBIO_Fn2* were able to protect the leaves from *B. cinerea.*
**Figure 4** shows *B. cinerea* was not inhibited in strawberry plants treated with application solution only (10 mM MgCl₂, 0.04% Silwet L-77 solution) and spread (circled in red) from the leaf disks collected from the Control plant group when placed on water agar plates. The fungus showed reduced spreading, or no spreading at all (circled in red), from the leaf disks collected from plants of the *PfBIO_Fn1* or *PfBIO_Fn2* groups.

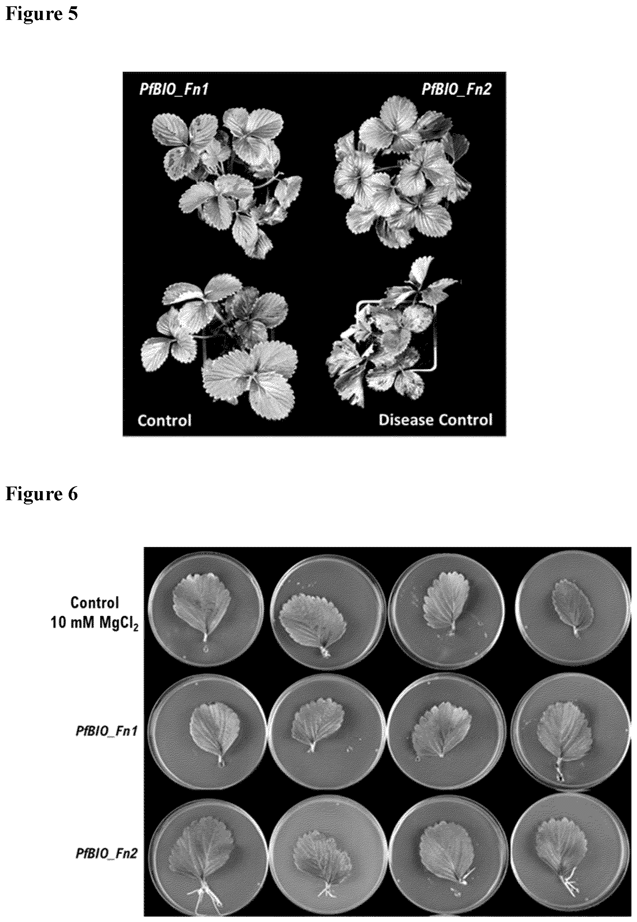

**Figure 5** shows a representative plant for each of the *PfBIO_Fn1, PfBIO_Fn2,* Control and Disease Control groups. Strawberry plants pre-treated with the single strains *PfBIO_Fn1* or *PfBIO_Fn2* showed mild or no crown rot symptoms, five weeks after being challenged with *P. cactorum.* In comparison, plants pre-treated with application solution only showed severe crown rot (Disease Control). Plants of the Control group were not challenged with the pathogens and did not show any disease symptoms.
**Figure 6** shows four representative strawberry leaves for each of the *PfBIO_Fn1, PfBIO_Fn2,* and Control groups. After 5 weeks in incubation in the dark and at room temperature, strawberry leaves of the group *PfBIO_Fn2* generated roots. In comparison, no root formation was observed from the strawberry leaves of the *PfBIO_Fn1* and Control groups.

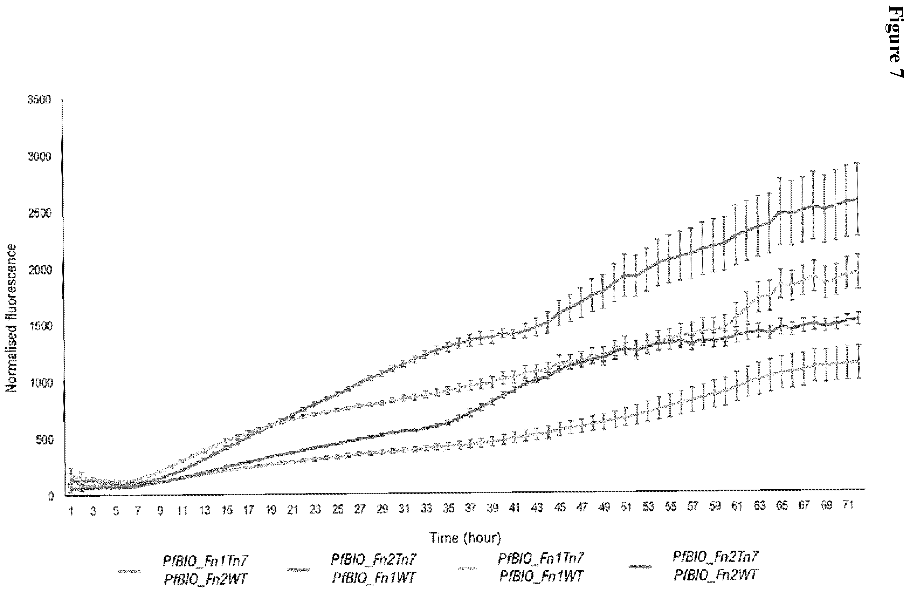

**Figure 7** shows representative growth curves for combinations:
   *PfBIO_Fn1Tn7*/*PfBIO_Fn2WT*;
   *PfBIO_Fn1WT*/*PfBIO_Fn2Tn7*;
   *PfBIO_Fn1Tn7*/*PfBIO_Fn1WT*; and
   *PfBIO_Fn2Tn7*/*PfBIO_Fn2WT.*
   Growth was measured in KB, at 28°C. Marked differences in growth rate were observed between *PfBIO_Fn1Tn7*/*PfBIO_Fn2WT* and *PfBIO_Fn1WT*/*PfBIO_Fn2Tn7.*

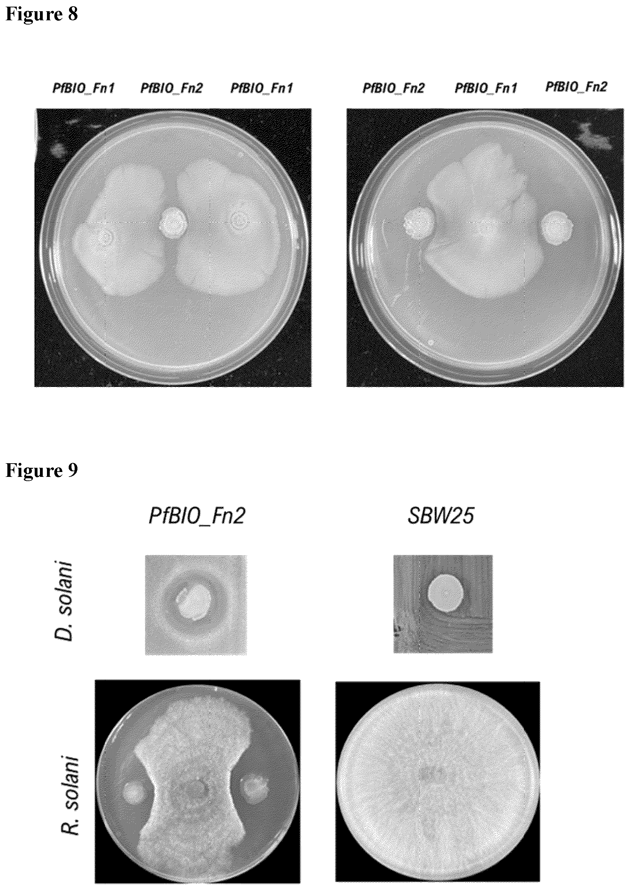

**Figure 8** shows inhibition assays performed on KB agar plates, incubated at 28°C for 5 days, to test the activity of the biocontrol strains, *PfBIO_Fn1* and *PfBIO_Fn2* respectively against each other. *PfBIO_Fn2* mildly inhibits growth of *PfBIO_Fn1* but both strains can co-occur without killing the other.
**Figure 9** shows inhibition assays performed on PDA media plates, incubated at 28°C up to 7 days, to test the activity of the biocontrol strain *PfBIO_Fn2* against *D. solani* (embedded in solid agar) and *R. solani* (plug placed in the middle of the plate). *PfBIO_Fn2* showed strong activity against the tested pathogens compared to the control strain, *Pseudomonas fluorescens* (SBW25).

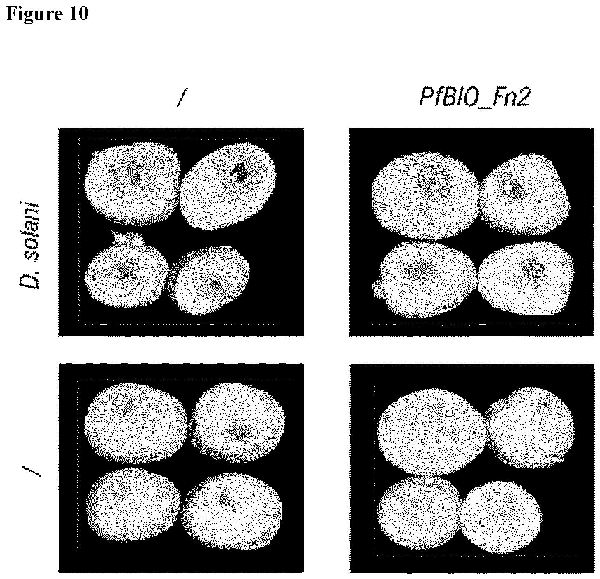

**Figure 10** Inhibition assays in potato tubers incubated at room temperature for five days to evaluate the activity of *PfBIO_Fn2* against *D. solani.* Control tubers, treated with either application solution or *PfBIO_Fn2* without *D. solani* challenge, showed no signs of infection. Similarly, tubers pre-treated with *PfBIO_Fn2* and subsequently challenged with *D. solani* remained infection-free. In contrast, tubers treated with application solution only and then challenged with *D. solani* exhibited signs of infection.

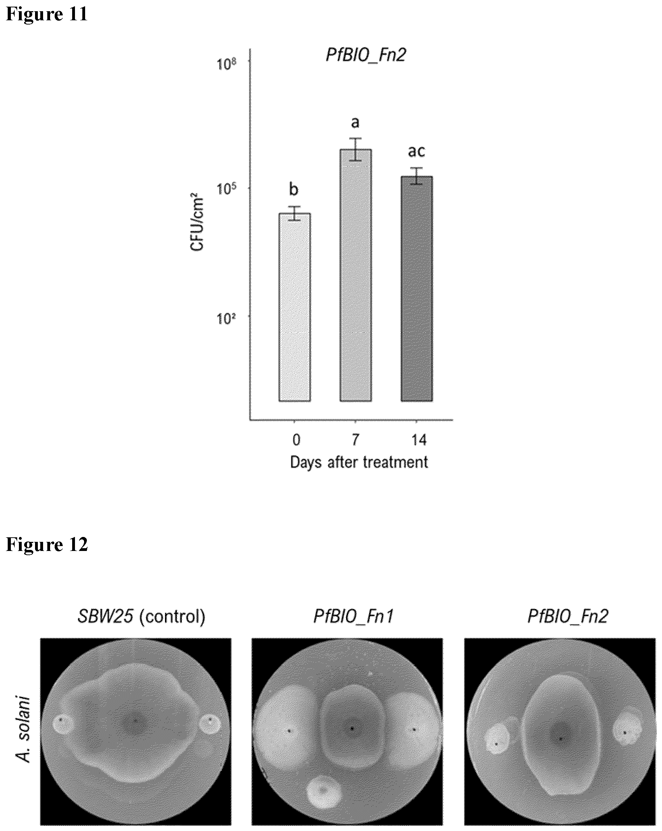

**Figure 11** shows the CFU counts for bacterial survival assays were performed on potato tubers placed in soil (pots) for 14 days. Samples were taken and processed after 0, 7 and 14 days. *PfBIO_Fn2* bacterial cells increased within the first week after treatment showing *PfBIO_Fn2* survives on potato tubers for at least 14 days.
**Figure 12** shows inhibition assays performed on PDA to test the activity of *PfBIO_Fn1* and *PfBIO_Fn2* against *A. solani.* Both *PfBIO_Fn1* and *PfBIO_Fn2* exhibited activity against the tested pathogen compared to the control strain, *Pseudomonas fluorescens* (SBW25).

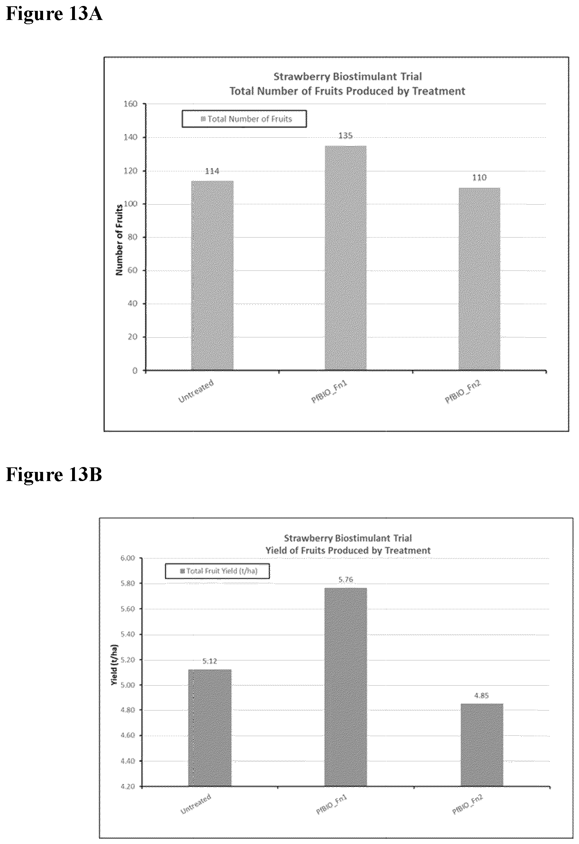

**Figure 13** shows the total number of fruits in Figure 13A and the yield of fruits in Figure 13B from strawberry plants, that were untreated or treated with *PfBIO_Fn1* or *PfBIO_Fn2* over the course of the experiment. The plants were grown under commercial conditions by a contractor specialising in agronomy, who conducted the trial.

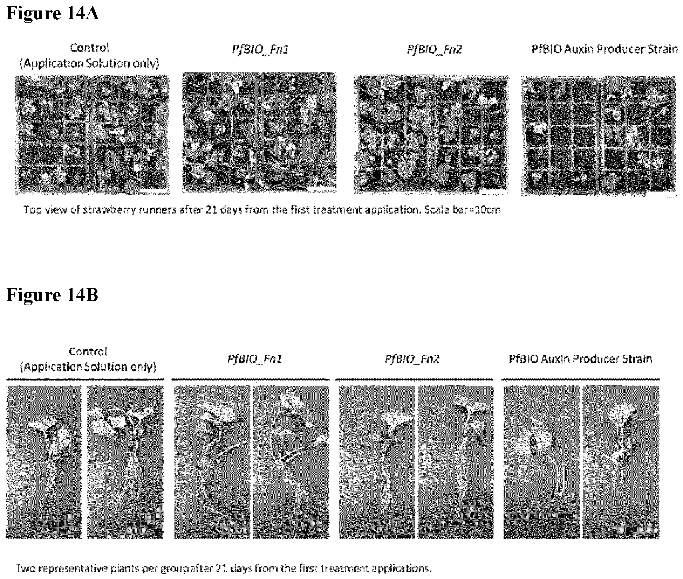

**Figure 14** shows the top view of strawberry runners after 21 days from the first treatment with control, *PfBIO_Fn1, PfBIO_Fn2,* or *PfBIO Auxin Producer strain* in Figure 14A. Scale bar=10 cm. Figure 14B shows two representative plants for each group 21 days after the first treatment.

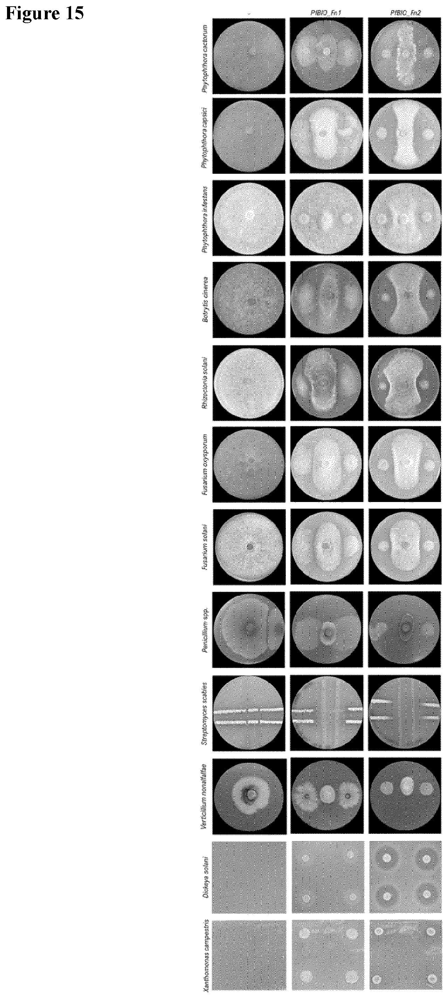

**Figure 15** shows inhibition assays performed on different growth media, appropriate for each specific pathogen, including PDA for *Phytophthora cactorum, P. capsici, Botrytis cinerea, Rhizoctonia solani, Fusarium oxysporum, F. solani, Penicillium* spp., *Verticillium nonalfalfae* and *Dickeya solani,* Rye-sucrose Agar (RSA) for *P. infestans,* Soya Flour and Mannitol (SF+M) for *Streptomyces scabies* and Lysogeny Broth (LB) for *Xanthomonas campestris.* The plates were incubated at 28°C for 7 days for *P. cactorum, P. capsici, B. cinerea, R. solani, F. oxysporum, F. solani, Penicillium* spp., and *S. scabies. V. nonalfalfae* and *P*. *infestans* were grown at room temperature for 14 days and 10 days, respectively. *D. solani* and *X. campestris* were grown at 28°C for 3 days. To evaluate the activity of the biocontrol strains *PfBIO_Fn1* and *PfBIO_Fn2* against various pathogens, including species of *Phytophthora, Botrytis, Rhizoctonia, Fusarium, Penicillium, Verticillium, Dickeya,* and *Xanthomonas,* these results were compared to a control using a microbe resuspension solution (10 mM MgCl₂). Both *PfBIO_Fn1* and *PfBIO_Fn2* exhibited strong activity against the tested pathogens, including fungi, oomycetes, and Gram-positive bacteria. Notably, only *PfBIO_Fn2* showed activity against Gram-negative bacteria.

### DETAILED DESCRIPTION

The invention provides a method of treating or preventing a eukaryotic and/or bacterial infection in a plant or promoting growth in a plant, the method comprising administering a *Pseudomonas* bacterium or extract thereof to the plant.

The method may be used to treat or prevent a eukaryotic infection, a bacterial infection, or both in a plant. The method may be used to promote growth in a plant, e.g. of the leaves, stem, fruit, roots and/or flowers of the plant. The method may be used to treat a eukaryotic and/or bacterial infection whilst simultaneously promoting growth in a plant. The method may comprise sequentially administering a *Pseudomonas* bacterium, or extract thereof, to the plant to first treat a eukaryotic and/or bacterial infection and then to promote growth in a plant. The method may comprise sequentially administering a *Pseudomonas* bacterium, or extract thereof, to the plant to first promote growth in a plant and then treat a eukaryotic and/or bacterial infection. The method may comprise multiple administrations of a *Pseudomonas* bacterium, or extract thereof, to a plant to alternate between treating a eukaryotic and/or bacterial infection and promoting growth in a plant. The method may comprise administering the *Pseudomonas* bacterium, or extract thereof, before there is a eukaryotic or bacterial infection, e.g. to prevent an infection of said plant.

The eukaryotic infection may be caused by a eukaryotic pathogen of the class *Oomycete.* The eukaryotic infection may be caused by a eukaryotic pathogen of the genus *Phytophthora.* The eukaryotic infection may be caused by a eukaryotic pathogen of the species *Phytophthora cactorum, Phytophthora infestans, Phytophthora capsici* and/or *Phytophthora plurivora.*

The eukaryotic infection may be caused by a fungal infection. The eukaryotic infection may be caused by a fungal pathogen of the genus *Rhizoctonia, Alternaria, Botrytis, Podosphaera, Fusarium, Penicillium* and/or *Verticillium.* The eukaryotic infection may be caused by a fungal pathogen of the species *Rhizoctonia solani, Alternaria solani, Botrytis cinerea, Fusarium oxysporum, Fusarium solani, Penicillium digitatum, Podosphaera aphanis,* and/or *Verticillium nonalfalfae.*

The bacterial infection may be caused by a bacterial pathogen of the genus *Pectobacterium, Streptomyces, Xanthomonas, Pseudomonas* and/or *Dickeya.* The bacterial infection may be caused by a bacterial pathogen of the species *Pectobacterium atrosepticum, Streptomyces scabies, Xanthomonas campestris* and/or *Dickeya solani.*

The *Pseudomonas* bacterium, or extract thereof, may be applied to the leaves, stems, flowers, fruits and/or roots of the plant. The application of the *Pseudomonas* bacterium to the leaves, stems, flowers, fruits and/or roots of the plant may occur multiple times as part of multiple administrations of the *Pseudomonas* bacterium. The application of the *Pseudomonas* bacterium may be conducted to different parts of the plants, i.e. the leaves, stems, flowers, fruits or roots of the plant, as part of multiple administrations of the *Pseudomonas* bacterium in the method. The application of the *Pseudomonas* bacterium may be conducted on different parts of the plants, i.e. the leaves, stems, flowers, fruits or roots of the plant, in order to target the *Pseudomonas* bacterium, or extract thereof, to a part of the plant experiencing a eukaryotic and/or bacterial infection or the part of the plant in need of growth promotion. The application of the *Pseudomonas* bacterium may be conducted to multiple parts of the plants, i.e. the leaves, stems, flowers, fruits and/or roots of the plant, in order to target the *Pseudomonas* bacterium, or extract thereof, to parts of the plant experiencing a eukaryotic and/or bacterial infection or the parts of the plant in need of growth promotion.

The *Pseudomonas* bacterium, or extract thereof, may be applied to the substrate surrounding the plant so that the *Pseudomonas* bacterium is administered to the plant. The substrate may be soil, compost, coir or an equivalent abiotic growth substrate, and/or an aqueous solution. The *Pseudomonas* bacterium, or extract thereof, may be applied to the substrate before the plant is positioned in the substrate. The *Pseudomonas* bacterium, or extract thereof, may be applied to the substrate once the plant is positioned in the substrate.

The *Pseudomonas* bacterium, or extract thereof, may be applied to both the leaves, stems, flowers, fruits and/or roots of the plant and the substrate surrounding the plant. The *Pseudomonas* bacterium, or extract thereof, may be applied simultaneously to the leaves, stems, flowers, fruits and/or roots of the plant and the substrate surrounding the plant. The *Pseudomonas* bacterium, or extract thereof, may be applied in alternative administrations to the leaves, stems, flowers, fruits and/or roots of the plant and then to the substrate surrounding the plant as part of multiple administrations of the *Pseudomonas* bacterium or extract thereof.

The *Pseudomonas* bacterium, or extract thereof, may be applied to the substrate surrounding the plant or directly applied to the plant as part of a composition. The composition may comprise more than one species of *Pseudomonas* bacterium. The composition may comprise further active ingredients and/or additional suitable excipients or carriers. The composition may be formulated as a liquid or a solid.

The composition may comprise *Pseudomonas* bacterium diluted to an OD₆₀₀ of 0.2. The composition may comprise *Pseudomonas* bacterium in a concentration of colony forming units (CFU) of 10⁸, 10⁹, 10¹⁰, 10¹¹ or 10¹² per ml. The composition may comprise *Pseudomonas* bacterium in a concentration of CFU 10¹¹ per ml. Routine methods for calculating cell density are known in the art, for example as described in Beal, J., *et al.* 2020 Commun Biol Vol. 3, Art. 512.

The composition may further comprise magnesium chloride, sodium chloride, magnesium citrate and/or phosphate buffered saline. The composition may comprise magnesium chloride at a concentration of 1-100 mM, 2-50 mM, 5-20 mM, 8-15 mM, 9-12 mM, or 10 mM MgCl₂. The composition may further comprise a surfactant. The composition may further comprise a silicone surfactant. The composition may further comprise Silwet L-77 (e.g. 0.01 - 0.1%, 0.02 - 0.08%, 0.03 - 0.06%, or 0.04% Silwet L-77). For example, the composition may comprise 10 mM MgCl₂ and/or 0.04% Silwet L-77.

The *Pseudomonas* bacterium, or extract thereof, may be applied to the substrate surrounding the plant or directly applied to the plant. The *Pseudomonas* bacterium or extract thereof may be applied to the substrate surrounding the plant or directly applied to the plant at intervals of at least every 3 days, every 5 days, every 7 days, every 14 days, every 21 days, every 4 weeks, every month, every 2 months and/or every 3 months. The *Pseudomonas* bacterium, or extract thereof, may be applied to the substrate surrounding the plant or directly applied to the plant over the course of 3 months, 6 months, 9 months, 12 months, 24 months and/or 36 months.

The *Pseudomonas* bacterium may have a 16S RNA gene with at least 80%, at least 85%, at least 90%, at least 95%, at least 98%, and/or at least 99% sequence identity with SEQ ID NOs 1 or 2. The *Pseudomonas* bacterium may have a 16S RNA gene with at least 90% sequence identity. The *Pseudomonas* bacterium may have a 16S RNA gene with at least 95% identity. The skilled person understands how to determine percentage sequence identity to a reference sequence using appropriate sequence alignments and analysis.

The *Pseudomonas* bacterium may be *PfBIO_Fn1* (accession number 507455) and/or *PfBIO_Fn2* (accession number 507456).

The *Pseudomonas* bacterium, or extract thereof, may be administered to the plant at intervals of at least every 3 days, every 5 days, every 7 days, every 14 days, every 21 days, every 4 weeks, every month, every 2 months and/or every 3 months. The *Pseudomonas* bacterium, or extract thereof, may be administered to the plant over the course of 3 months, 6 months, 9 months, 12 months, 24 months and/or 36 months. The *Pseudomonas* bacterium, or extract thereof, may be administered to the plant over the plant's lifetime. The *Pseudomonas* bacterium, or extract thereof, may be administered to the plant only once.

The administration of the *Pseudomonas* bacterium, or extract thereof, may not adversely impact the health and/or growth of the plant. Health of the plant may be determined by colour and/or form of the leaves, stems, flowers, fruits and/or roots of the plant. Growth of the plant may be determined by measurements of length of parts of the plants, i.e. length of the stems, length of the flowers, length of the fruits and/or length of the roots. Growth of the plant may be determined by measurements of the mass of the plant or parts of the plants, i.e. mass of the flowers, mass of the fruits and/or mass of the seeds.

The invention provides a method of producing a *Pseudomonas* extract, the method comprising:
a) culturing a *Pseudomonas* bacterium under suitable conditions;
b) soaking the *Pseudomonas* culture in an extraction solution;
c) filtering the *Pseudomonas* culture and extraction solution;
d) drying the *Pseudomonas* filtrate; and
e) resuspending the *Pseudomonas* filtrate.

Culturing a *Pseudomonas* bacterium under suitable conditions may comprise incubating the *Pseudomonas* bacterium at 28 °C. Culturing a *Pseudomonas* bacterium under suitable conditions may comprise incubating the *Pseudomonas* strain in suitable culture media with shaking over a suitable period of time and at a suitable temperature. Culturing a *Pseudomonas* bacterium under suitable conditions may comprise incubating the *Pseudomonas* strain in a liquid medium at a suitable temperature (e.g. 22-34°C, 24-32°C, 26-30°C, 27-29°C or 28°C) for a suitable period of time (e.g. for 5-70 hours, 10-65 hours, 12-60 hours, 14-55 hours, 16-50 hours, or 24-48 hours). Culturing a *Pseudomonas* bacterium under suitable conditions may comprise incubating the *Pseudomonas* strain in King's B (KB) liquid medium at 28°C with 250 rpm shaking overnight. Culturing a *Pseudomonas* bacterium under suitable conditions may comprise culturing the *Pseudomonas* strain in an aerobic fermenter, e.g. for 5-70 hours, 10-65 hours, 12-60 hours, 14-55 hours, 16-50 hours, or 24-48 hours. For example, culturing a *Pseudomonas* bacterium under suitable conditions may comprise culturing in King's B (KB) liquid medium at 28°C in an aerobic fermenter for 24 hours or 48 hours.

The extraction solution may comprise methanol, ethyl acetate, and/or acetonitrile. For example, the extraction solution may be 40% v/v methanol in water, 50% v/v methanol in water or 60% v/v methanol in water or 75% v/v methanol in water. The extraction solution may be 80% v/v ethyl acetate in water, 90% v/v ethyl acetate, 95% v/v ethyl acetate in water of 100% ethyl acetate. The extraction solution may be 40% v/v acetonitrile in water, 50% v/v acetonitrile in water or 60% v/v acetonitrile in water.

### Definitions

In order that the present disclosure can be more readily understood, certain terms and methodologies are first defined. As used in this application, except as otherwise expressly provided herein, each of the following terms shall have the meaning set forth below. Additional definitions are set forth throughout the application. In case of conflict, the present application including the definitions will control. Unless otherwise required by context, singular terms shall include pluralities and plural terms shall include the singular. All publications, patents and other references mentioned herein are incorporated by reference in their entireties for all purposes as if each individual publication or patent application were specifically and individually indicated to be incorporated by reference.

Unless defined otherwise, all technical and scientific terms used herein have the same meaning as commonly understood by one of ordinary skill in the art to which this disclosure is related.

Although methods and materials similar or equivalent to those described herein can be used in practice or testing of the present disclosure, suitable methods and materials are described below. The materials, methods and examples are illustrative only and are not intended to be limiting. Other features and advantages of the disclosure will be apparent from the detailed description and from the claims.

As used in the present disclosure and claims, the singular forms *"a", "an",* and *"the"* include plural forms unless the context clearly dictates otherwise. As an example, *"an element"* may mean one element or more than one element, i.e. *"at least one element".*

For the descriptions herein and the appended claims, the use of *"or"* means *"and*/*or"* unless stated otherwise. Similarly, *"comprise", "comprises", "comprising", "include", "includes",* and *"including"* are interchangeable and not intended to be limiting.

It is to be further understood that where descriptions of various embodiments use the term *"comprising"* those skilled in the art would understand that in some specific instances, an embodiment can be alternatively described using language *"consisting essentially of"* or *"consisting of".*

The terms *"purified", "substantially purified',* and *"isolated"* as used herein refer to the state of being free of other, dissimilar compounds with which a compound of the invention is normally associated in its natural state, so that the *"purified", "substantially purified',* and *"isolated"* subject comprises at least 0.5%, 1%, 5%, 10%, or 20%, or at least 50% or 75% of the mass, by weight, of a given sample. In one embodiment, these terms refer to the compound of the invention comprising at least 95, 96, 97, 98, 99 or 100%, of the mass, by weight, of a given sample. As used herein, the terms *"purified", "substantially purified",* and *"isolated"* when referring to a nucleic acid or protein, or nucleic acids or proteins, also refers to a state of purification or concentration different than that which occurs naturally, for example in a prokaryotic or eukaryotic environment, like, for example in a bacterial or fungal cell, or in the mammalian organism, especially human body. Any degree of purification or concentration greater than that which occurs naturally, including (1) the purification from other associated structures or compounds or (2) the association with structures or compounds to which it is not normally associated in said prokaryotic or eukaryotic environment, are within the meaning of *"isolated".* The nucleic acid or protein or classes of nucleic acids or proteins, described herein, may be isolated, or otherwise associated with structures or compounds to which they are not normally associated in nature, according to a variety of methods and processes known to those of skill in the art.

The term *"about"* indicates a potential variation of ± 25% of the stated value, in particular ± 15%, ± 10 %, more particularly ± 5%, ± 2% or ± 1%.

The term *"substantially"* describes a range of values of from about 80 to 100%, such as, for example, 85-99.9%, in particular 90 to 99.9%, more particularly 95 to 99.9%, or 98 to 99.9% and especially 99 to 99.9%.

The terms *"bacteria", "bacterium, "bacterial"* can be used interchangeably to refer to single-cell microorganisms belonging to the prokaryotic kingdom.

The term *"bacterial infection"* encompasses infections caused by any bacteria.

The term *"biostimulant"* refers to the ability to induce growth in the plant. Growth can be induced in one or more parts of the plant including leaves, stem, fruit, roots and/or flowers.

The term *"eukaryotic"* refers to single-cell and multi-cellular organisms that contain a nucleus and membrane-bound organelles.

The term *"eukaryotic infection"* encompasses infections caused by any eukaryotic organism, including fungi and oomycetes.

The terms *"fungi", "fungus", "fungal"* can be used interchangeably to refer to single-cell and multi-cellular eukaryotic organisms that have cell walls comprising chitin.

The term *"inhibition"* refers to the ability to prevent or slow down growth of a microorganism.

The term *"auxin-producer"* refers to a *Pseudomonas* strain capable of the biosynthesis of the plant growth hormone indole-3-acetic acid (IAA, auxin).

The term *"rooting success"* describes the survival rate of plants potted with no visible root, for example strawberry runners with no roots or propagated cutting. Thus, the survival of plants is due to the ability to form roots that then sustain the plant.

### INFORMAL SEQUENCE LISTING

***PfBIO_Fn1* (accession number 507455) 16s RNA gene sequence**
   **SEQ ID NO: 1** 
GAACTGAAGAGTTTGATCATGGCTCAGATTGAACGCTGGCGGCAGGCCTAACAC
ATGCAAGTCGAGCGGTAGAGAGAAGCTTGCTTCTCTTGAGAGCGGCGGACGGGT
GAGTAATGCCTAGGAATCTGCCTGGTAGTGGGGGATAACGTTCGGAAACGGACG
CTAATACCGCATACGTCCTACGGGAGAAAGCAGGGGACCTTCGGGCCTTGCGCTA
TCAGATGAGCCTAGGTCGGATTAGCTAGTTGGTGAGGTAATGGCTCACCAAGGCG
ACGATCCGTAACTGGTCTGAGAGGATGATCAGTCACACTGGAACTGAGACACGG
TCCAGACTCCTACGGGAGGCAGCAGTGGGGAATATTGGACAATGGGCGAAAGCC
TGATCCAGCCATGCCGCGTGTGTGAAGAAGGTCTTCGGATTGTAAAGCACTTTAA
GTTGGGAGGAAGGGCATTAACCTAATACGTTAGTGTTTTGACGTTACCGACAGAA
TAAGCACCGGCTAACTCTGTGCCAGCAGCCGCGGTAATACAGAGGGTGCAAGCG
TTAATCGGAATTACTGGGCGTAAAGCGCGCGTAGGTGGTTTGTTAAGTTGGATGT
GAAATCCCCGGGCTCAACCTGGGAACTGCATTCAAAACTGACTGACTAGAGTATG
GTAGAGGGTGGTGGAATTTCCTGTGTAGCGGTGAAATGCGTAGATATAGGAAGG
AACACCAGTGGCGAAGGCGACCACCTGGACTAATACTGACACTGAGGTGCGAAA
GCGTGGGGAGCAAACAGGATTAGATACCCTGGTAGTCCACGCCGTAAACGATGT
CAACTAGCCGTTGGAAGCCTTGAGCTTTTAGTGGCGCAGCTAACGCATTAAGTTG
ACCGCCTGGGGAGTACGGCCGCAAGGTTAAAACTCAAATGAATTGACGGGGGCC
CGCACAAGCGGTGGAGCATGTGGTTTAATTCGAAGCAACGCGAAGAACCTTACC
AGGCCTTGACATCCAATGAACTTTCTAGAGATAGATTGGTGCCTTCGGGAACATT
GAGACAGGTGCTGCATGGCTGTCGTCAGCTCGTGTCGTGAGATGTTGGGTTAAGT
CCCGTAACGAGCGCAACCCTTGTCCTTAGTTACCAGCACGTAATGGTGGGCACTC
TAAGGAGACTGCCGGTGACAAACCGGAGGAAGGTGGGGATGACGTCAAGTCATC
ATGGCCCTTACGGCCTGGGCTACACACGTGCTACAATGGTCGGTACAGAGGGTTG
CCAAGCCGCGAGGTGGAGCTAATCCCATAAAACCGATCGTAGTCCGGATCGCAG
TCTGCAACTCGACTGCGTGAAGTCGGAATCGCTAGTAATCGCGAATCAGAATGTC
 
GCGGTGAATACGTTCCCGGGCCTTGTACACACCGCCCGTCACACCATGGGAGTGG
GTTGCACCAGAAGTAGCTAGTCTAACCTTCGGGAGGACGGTTACCACGGTGTGAT
TCATGACTGGGGTGAAGTCGTAACAAGGTAGCCGTAGGGGAACCTGCGGCTGGA
TCACCTCCTTA
***Pseudomonas PfBIO_Fn2* (accession number 507456) 16s RNA gene sequence**
   **SEQ ID NO: 2**

GAACTGAAGAGTTTGATCATGGCTCAGATTGAACGCTGGCGGCAGGCCTAACAC
ATGCAAGTCGAGCGGTAGAGAGGTGCTTGCACCTCTTGAGAGCGGCGGACGGGT
GAGTAATGCCTAGGAATCTGCCTGGTAGTGGGGGATAACGCTCGGAAACGGACG
CTAATACCGCATACGTCCTACGGGAGAAAGCAGGGGACCTTCGGGCCTTGCGCTA
TCAGATGAGCCTAGGTCGGATTAGCTAGTTGGTGGGGTAATGGCTCACCAAGGCG
ACGATCCGTAACTGGTCTGAGAGGATGATCAGTCACACTGGAACTGAGACACGG
TCCAGACTCCTACGGGAGGCAGCAGTGGGGAATATTGGACAATGGGCGAAAGCC
TGATCCAGCCATGCCGCGTGTGTGAAGAAGGTCTTCGGATTGTAAAGCACTTTAA
GTTGGGAGGAAGGGCATTAACCTAATACGTTAGTGTTTTGACGTTACCGACAGAA
TAAGCACCGGCTAACTCTGTGCCAGCAGCCGCGGTAATACAGAGGGTGCAAGCG
TTAATCGGAATTACTGGGCGTAAAGCGCGTAGGTGGTTCGTTAAGTTGGATGT
GAAATCCCCGGGCTCAACCTGGGAACTGCATTCAAAACTGTCGAGCTAGAGTATG
GTAGAGGGTGGTGGAATTTCCTGTGTAGCGGTGAAATGCGTAGATATAGGAAGG
AACACCAGTGGCGAAGGCGACCACCTGGACTGATACTGACACTGAGGTGCGAAA
GCGTGGGGAGCAAACAGGATTAGATACCCTGGTAGTCCACGCCGTAAACGATGT
CAACTAGCCGTTGGGAGCCTTGAGCTCTTAGTGGCGCAGCTAACGCATTAAGTTG
ACCGCCTGGGGAGTACGGCCGCAAGGTTAAAACTCAAATGAATTGACGGGGGCC
CGCACAAGCGGTGGAGCATGTGGTTTAATTCGAAGCAACGCGAAGAACCTTACC
AGGCCTTGACATCCAATGAACTTTCCAGAGATGGATTGGTGCCTTCGGGAACATT
GAGACAGGTGCTGCATGGCTGTCGTCAGCTCGTGTCGTGAGATGTTGGGTTAAGT
CCCGTAACGAGCGCAACCCTTGTCCTTAGTTACCAGCACGTAATGGTGGGCACTC
TAAGGAGACTGCCGGTGACAAACCGGAGGAAGGTGGGGATGACGTCAAGTCATC
ATGGCCCTTACGGCCTGGGCTACACACGTGCTACAATGGTCGGTACAGAGGGTTG
CCAAGCCGCGAGGTGGAGCTAATCCCACAAAACCGATCGTAGTCCGGATCGCAG
TCTGCAACTCGACTGCGTGAAGTCGGAATCGCTAGTAATCGCGAATCAGAATGTC
GCGGTGAATACGTTCCCGGGCCTTGTACACACCGCCCGTCACACCATGGGAGTGG

GTTGCACCAGAAGTAGCTAGTCTAACCTTCGGGGGGACGGTTACCACGGTGTGAT
TCATGACTGGGGTGAAGTCGTAACAAGGTAGCCGTAGGGGAACCTGCGGCTGGA
TCACCTCCTTA

### EXAMPLES

### Example 1 - Identification of Pseudomonas PfBIO_Fn1 and Pseudomonas PfBIO_Fn2

2000 bacterial strains were isolated from commercial strawberry farms. These isolated strains were then screened for their ability to inhibit *in vitro* the growth of both *B. cinerea spp.* and the oomycete *P. cactorum.* Inhibition assays were performed by incubating *PfBIO_Fn1* and *PfBIO_Fn2* overnight in KB liquid medium at 28°C with 250 rpm shaking. The following day, the bacterial cultures were washed twice in 10 mM MgCl₂ and diluted to an OD₆₀₀ of 0.2. 5 µl of the diluted bacterial culture was then added to an agar plate containing a pathogen plug in the centre. Plates were then incubated at 28°C and monitored up to 7 days. The *Pseudomonas fluorescens SBW25* strain was used as a negative control (Figure 1).

### Example 2 - Generation of transgenic Pseudomonas PfBIO_Fn1 and Pseudomonas PfBIO_Fn2 strains

Following the protocol described in Choi & Schweizer, Nature Protocols 2006, a Tn7 vector carrying both a gentamicin resistance cassette and an mCherry reporter gene were introduced into *PfBIO_Fn1* and *PfBIO_Fn2.* Following transformation, *PfBIO_Fn1* and *PfBIO_Fn2* strain were grown on gentamycin selective plates. Further screening was conducted by PCR using a pair of primers designed to recognise the Tn7 sequence (internal) and the *Pseudomonas* genome sequence adjacent to the transposon insertion point (external), and by measuring the fluorescence due to the presence of the mCherry tag using a fluorescence microplate reader.

### Example 3 - Survival test of Pseudomonas PfBIO_Fn1 and Pseudomonas PfBIO_Fn2 strains

The transgenic bacterial strains generated in accordance with Example 2 were applied in the range of CFU of 10⁸ to strawberry plants by spraying leaves and soaking roots. Strawberry plants were grown in coir at 15-20°C in 70% humidity.

The ability of *PfBIO_Fn1* and *PfBIO_Fn2* to survive on plant leaves and roots was assessed at specific time points (Day 0, Day 7, Day 14, and Day 28 post administration). Survival of *PfBIO_Fn1* and *PfBIO_Fn2* on both strawberry plant leaves and roots was confirmed for at least two weeks post-administration (Figure 2). These data demonstrate the ability of *PfBIO_Fn1* and *PfBIO_Fn2* to survive in commercial settings.

### Example 4 - Protection assay for Botrytis cinerea leaf infections

Strawberry leaves were first sterilised using 1% sodium hypochlorite solution and then divided into three groups: *PfBIO_Fn1, PfBIO_Fn2,* and Disease Control. *PfBIO_Fn1* and *PfBIO_Fn2* groups were each treated with the respective *Pseudomonas* strain resuspended in 10mM MgCl₂ and 0.04% Silwet L-77. The Disease Control, were also treated with 10mM MgCl₂ and 0.04% Silwet L-77 solution (application solution). Each treatment was applied to strawberry leaves by soaking the leaves for 30 minutes in a bacterial culture having an OD₆₀₀ of 0.2 (CFU in the 10⁸/ml range), or application solution only. The leaves were dried for 45 minutes then placed on water agar plates. Fungal plugs generated from a *Botrytis cinerea* PDA plate were then placed on top of each leaf. The leaves were incubated in the dark, at room temperature for two weeks to assess the progression of the fungal infection. The results obtained show how both the selected strains can strongly suppress fungus spreading and protect the strawberry leaves from infection as shown by the lack of tissue death (Figure 3).

### Example 5 - Protection assay for Botrytis cinerea whole plant infections

*Botrytis* symptoms do not necessarily appear on plant leaves. Usually, the *Botrytis cinerea* infection starts on the flowers or fruits. To assess the preventative effects for *Botrytis cinerea* whole plant infections, strawberry plants were divided into three groups: *PfBIO_Fn1, PfBIO_Fn2,* and Control. *PfBIO_Fn1* and *PfBIO_Fn2* groups were each treated with the respective *Pseudomonas* strain resuspended in 10 mM MgCl₂ and 0.04% Silwet L-77 in the range of CFU 10⁸/ml. Control group was sprayed with application solution only (10 mM MgCl₂ and 0.04% Silwet L-77). After a week, all groups were resprayed in the same way with the same solution as administered in the first treatment. Each group was then challenged with *Botrytis cinerea* spores (2 x 10⁶/ ml). After 24 hours, leaf disks were collected from each plant, placed on water agar plates, and incubated at 28°C to examine the fungus' presence on the leaves. If *Botrytis cinerea* was present on the leaf disks, it was expected that mycelia would be observed spreading through the water agar plates. As shown in Figure 4, fungal mycelia were only observed from the leaf disks collected from the Control group. These data indicate a strong ability for *PfBIO_Fn1* and *PfBIO_Fn2* to prevent *Botrytis cinerea* infections.

### Example 6 - Protection assay for Phytophthora cactorum whole plant infections

The ability of the strains *PfBIO_Fn1* and *PfBIO_Fn2* to protect strawberry plants from *Phytophthora cactorum* infections was tested. *PfBIO_Fn1* and *PfBIO_Fn2 Pseudomonas* strains were individually grown overnight in KB medium at 28°C. The following day, the overnight cultures were washed twice in 10 mM MgCl₂ and resuspended in 10 mM MgCl₂ and 0.04% Silwet L-77.

Strawberry plants were grown in a controlled environmental cabinet at 20°C during daytime and 15°C during nighttime, 70% humidity, with a 16-hour photoperiod. The plants were divided into four groups, and each group was treated with their respective treatments as showed in the table below.

| **Group** | **Treatment** | **Pathogen Inoculation** |
|---|---|---|
| A | *PfBIO_Fn1* (CFU 10¹²/L) | Yes |
| B | *PfBIO_Fn2* (CFU 10¹²/L) | Yes |
| C (Disease Control) | 10 mM MgCl₂ and 0.04% Silwet L-77 | Yes |
| D (Control) | 10 mM MgCl₂ and 0.04% Silwet L-77 | No |

The treatments were applied twice by 30-minute soaking, with an interval of 7 days between each treatment. The day after the second application, groups A, B, and C were challenged with 2 × 10⁵ zoospores *P. cactorum* in application solution. Group D was treated with an equal volume of application solution only.

Subsequently, the presence of crown infections was followed over a 5-week period and the number of disease plants recorded. After 5 weeks, the plants of the Disease Control group were all severely affected by *P. cactorum,* with 100% of plants showing symptoms. In contrast, only 10% of plants in group A and 20% of plants in group B showed symptoms of infection. The Control plants were all symptom-free (Figure 5). This experiment demonstrates that the biocontrol strains can effectively prevent *P. cactorum* infections in strawberry plants.

### Example 7 - Assay for biostimulant activity

Strawberry leaves were first sterilised using 1% sodium hypochlorite, and then divided into three groups: *PfBIO_Fn1, PfBIO_Fn2,* and Control. *PfBIO_Fn1* and *PfBIO_Fn2* groups were each treated with the respective *Pseudomonas* strain resuspended in 10 mM MgCl₂ and 0.04% Silwet L-77. Control groups were treated with application solution only (10 mM MgCl₂ and 0.04% Silwet L-77). The treatments were applied by soaking the leaves for 30 minutes in the respective *Pseudomonas* culture having an OD₆₀₀ of 0.2 (CFU in the 10⁸/ml range), or in application solution only. The leaves were left to dry for 45 minutes and then placed on water agar plates. After 4 weeks, it was observed that only the leaves treated with *PfBIO_Fn2* strain showed root formation from leaves (Figure 6). These data indicate that *PfBIO Fn2* has biostimulant properties, particularly relating to root formation.

### Example 8 - Co-administration of Pseudomonas PfBIO_Fn1 and Pseudomonas PfBIO_Fn2

To determine if *Pseudomonas PfBIO_Fn1* and *Pseudomonas PfBIO_Fn2* can be co-administered, both growth assays for coculturing and inhibition assays were conducted.

The growth assay was performed by using a combination of the strains carrying the Tn7 Gm mCherry (Tn7) described in Example 2 and their corresponding wild type (WT) strains. Bacterial growth was monitored in a fluorescent microplate spectrophotometer with a minimum of eight experimental replicates/sample. A 96-well plate containing 135 µL of KB growth medium per well was used for each experiment. Growth was initiated by the addition of 15µl of the appropriate cell culture combinations (7.5 µl of each strain listed in the table below), with an OD₆₀₀ of 0.01. Plates were covered with adhesive sealing sheets and incubated at 28°C with moderate shaking. OD₆₀₀ and mCherry fluorescence was measured every hour over a period of 3 days.

| **Group** | **Strain 1** | **Strain 2** |
|---|---|---|
| Mixed | *Pseudomonas PfBIO_Fn1 Tn7* | *Pseudomonas PfBIO_Fn2* |
| Mixed | *Pseudomonas PfBIO_Fn2 Tn7* | *Pseudomonas PfBIO_Fn1* |
| Single | *Pseudomonas PfBIO_Fn1 Tn7* | *Pseudomonas PfBIO_Fn1* |
| Single | *Pseudomonas PfBIO_Fn2 Tn7* | *Pseudomonas PfBIO_Fn2* |

Both single strain groups showed comparable growth rates. However, in the mixed groups, it was observed that *PfBIO_Fn2* has a growth advantage over *PfBIO_Fn1* (Figure 7).

The inhibition assays were performed by spotting a central dot of one *Pseudomonas* strain on an agar plate and then spotting on the opposite side of the central dot the other *Pseudomonas* strain. Aliquots of 10 µL of the single WT strains having an OD₆₀₀ of 0.2 on KB agar plates were used for each respective spot. The plates were then incubated at 28°C and monitored for a period of 5 days. The results of these assays indicate that *PfBIO_Fn1* spread over the plate at faster rate than *PfBIO_Fn2.* Moreover, *PfBIO_Fn2* slightly inhibits growth of *PfBIO_Fn1* (Figure 8).

*PfBIO_Fn2* mildly inhibits the growth of *PfBIO_Fn1,* whilst *PfBIO_Fn1* exhibits a faster growth rate in monoculture than *PfBIO_Fn2.* The combination of these two *Pseudomonas* strain behaviours is advantageous for the survival of both strains when co-administered.

### Example 9 - Inhibition of Dickeya solani growth and Rhizoctonia solani growth

The inhibition assays were performed for *Dickeya solani,* a pathogen responsible for blackleg in potatoes and various rot diseases in other plant species.

The inhibition assay was conducted by embedding *D. solani* centrally in PDA media plates and then spotting on opposite side with *Pseudomonas PfBIO_Fn2.* Plates were then incubated at 28°C for 7 days.

The inhibition assays were performed for the fungus *Rhizoctonia solani,* a pathogen know for damping off stem lesions, stem rot, root rot, crown rot, and aerial web blight.

The inhibition assay was conducted by placing a *R. solani* plug centrally on PDA media plates and then spotting on opposite sides with 5 µL *Pseudomonas PfBIO_Fn2.* Plates were then incubated at 28°C for 7 days.

These data demonstrate that *PfBIO_Fn2* has both bactericide and fungicidal activities.

### Example 10 - Survival of Pseudomonas PfBIO_Fn2 on potato tubers

The Tn7 version of *Pseudomonas PfBIO_Fn2* carrying a gentamicin resistance and mCherry fluorescent cassette was tracked to check the survival of the strain on potato tubers over a two-week period. Potato tubers were immersed in a *Pseudomonas PfBIO_Fn2* culture resuspended in 10 mM MgCl₂ and 0.04% Silwet L-77, having density of OD₆₀₀ 0.2 (CFU 10⁸/ml range) for 2 hours. The control group of potatoes was immersed in application solution only (10mM mgCl₂ and 0.04% Silwet L-77). Potato tubers were then left to dry at room temperature for 30 minutes.

The t0 timepoint samples were then collected immediately after the drying step. The remaining potato tubers in each group were placed in pots containing soil and incubated in a controlled environmental cabinet (Temperature: 15°C during the day, 12°C at night. Humidity: 70% during the day, 60% at night. Photoperiod: 16h). To collect samples, potatoes were peeled, and 12 disks from the peels of each potato were collected and divided in three in tubes containing 1 mL of 10 mM MgCl₂ and two glass beads. The tubes were vigorously vortexed for 1 minute. The suspensions were then serially diluted and then spotted on Gm CFC *Pseudomonas* selective medium plates. The plates were incubated at 28°C and monitored to count the emerging colonies. This step was repeated at day 7 and day 14. It was observed that the *PfBIO_Fn2* strain was able to survive on potato tubers for an extended time period.

The obtained results indicate that *Pseudomonas PfBIO_Fn2* not only survives on potato tubers but continues to proliferate within the first week after application. These data also suggest that *PfBIO_Fn2* is able to survive on potato tubers for at least 14 days, with the potential for longer term survival (Figure 11).

### Example 11 - PfBIO_Fn1 and PfBlO_Fn2 inhibit A. solani growth

The inhibition assays were performed for fungus *A. solani,* a pathogen known for causing early blight in potatoes.

The inhibition assay was conducted by placing an *A. solani* plug centrally on PDA medium plates and then spotting 5 µL of *Pseudomonas* culture on opposite sides with *Pseudomonas PfBIO_Fn2.* Plates were then incubated at 28°C for 7 days.

### These data show that both PfBIO_Fn1 and PfBIO_Fn2 strains inhibit growth of the fungal pathogen A. solani (Figure 12).

### Example 13 - PfBIO_Fn1 and PfBIO_Fn2 inhibit P. infestans growth

The inhibition assays were performed for oomycete *P. infestans,* a pathogen known for causing late blight in potatoes.

The inhibition assay was conducted by placing a *P. infestans* plug centrally on rye media plates and then spotting 5 µL of *Pseudomonas* culture on the opposite sides with *Pseudomonas PfBIO_Fn1 and PfBIO_Fn2.* Plates were then incubated at room temperature for 7 days.

These data show that both *PfBIO_Fn1* and *PfBIO_Fn2* strains inhibit the growth of the pathogen *oomycete P. infestans* (Figure 15).

### Example 14 - Pseudomonas PfBIO_Fn1 and Pseudomonas PfBIO_Fn2 extracts inhibit fungal growth

Both *Pseudomonas PfBIO_Fn1* and *Pseudomonas PfBIO_Fn2* were grown on two different growth media: PDA plates and KB plates. *Pseudomonas PfBIO_Fn1* and *Pseudomonas PfBIO_Fn2* were grown for 6 days at 28°C. After this time, the plates were divided into two groups and processed: cut into small pieces and soaked in one of three different extraction solutions: 50% methanol, Ethyl acetate or 50% acetonitrile.

The first group was processed on the same day, after 2 hours of soaking in the respective solvents.

The second group was left soaking overnight at 4°C and processed the day after. Following this, the extracts were filtered and centrifuged, and then dried using a GeneVac and further resuspended in 50% methanol (1 mL) for activity test against pathogen (*Botrytis cinerea*) on PDA plates.

The crude extracts of *PfBIO_Fn1* and *PfBIO_Fn2* (grown in PDA or KB media), using 50% methanol extracting solvent, strongly inhibit fungal growth, especially if the extraction process was extended overnight at 4°C. This indicates that the process used worked well for the extraction of bioactive molecules from *Pseudomonas PfBIO_Fn1* and *Pseudomonas PfBIO_Fn2.*

### Example 15 - Evaluation of Pseudomonas PfBIO_Fn1 and Pseudomonas PfBIO_Fn2 biostimulant properties in strawberry fruiting

To evaluate the biostimulant effects of *Pseudomonas PfBIO_Fn1* and *Pseudomonas PfBIO_Fn2* on strawberry fruiting, a suspension of approximately 1 × 10¹² CFU/L of each strain was applied to strawberry plants. An untreated control group, receiving no bacterial treatment, was included for comparison. The trial conducted under commercial conditions by a contractor specializing in agronomy, included five replicates per treatment, with each plot containing eight plants, and each plot was supplemented with one bag of coir (1 metre in length). The Malling Vitality strawberry variety was used, and the plots were randomised to ensure unbiased results. Treatments were applied via soil drench, with two applications spaced one month apart, before the plants reached their senescent period. The trial results showed an approximately 18% increase in fruiting for plants treated with strain *PfBIO_Fn1,* demonstrating the potential of this biostimulant to enhance strawberry yield (see Figure 13A and 13B).

### Results

| **Treatment name** | **Rate (l/ha)** | **Total Number of Fruits** | **Total Fruit Yield (t/ha)** |
|---|---|---|---|
| Untreated | - | 114 | 5.12 |
| *PfBIO_Fn1* | 1.0 | 135 | 5.76 |
| *PfBIO_Fn2* | 1.0 | 110 | 4.85 |

### Example 16 - Evaluation of Pseudomonas PfBIO_Fn1 and Pseudomonas PfBIO_Fn2 biostimulant properties in strawberry rooting

30 strawberry runners of the Malling Centenary variety were treated weekly with application solution only (10mM MgCl₂, 0.04% Silwet L-77) or *Pseudomonas PfBIO_Fn1, Pseudomonas PfBIO_Fn2* or a *Pseudomonas* auxin-producer strain. The experiment was repeated three times according to the methodology below.

### Methodology

1) Strawberry runners were selected for a similar development stage for each group, then placed in 3 x 5 square tray pots filled with washed coir. Two trays of 15 plants were potted for each group.
*2) Pseudomonas* cultures were grown in 200 mL KB at 28°C overnight at 250 rpm, before washing three times in 10 mM MgCl₂.
3) Cells were diluted to OD₆₀₀ 0.2 (in the range of 10⁸) for *PfBIO_Fn1, PfBIO_Fn2* and the PfBIO auxin-producer, respectively, with 0.04% Silwet L-77. 10 ml of cell suspensions were added to each pot and applied weekly for 3 weeks.
4) Plants were incubated in a plant-controlled environmental room (25°C, with a 16-hour photoperiod).
5) Each tray (2 x 15 plants) was watered regularly over the course of the experiments to prevent drying out and stress to the plants.
6) Rooting was checked weekly and photographed on soil before re-planting and application of treatments.
7) Final washing and photography took place on day 21, with roots cut into individual Petri dishes and dried at 60°C for 2 days.
8) Root weights were measured on an analytical scale.

### Results

After 3 weeks, 30 strawberry runners of the Malling Centenary variety (2 x 15 pots) were assessed for rooting success with each treatment (Figures 14A and 14B).

The average dry root weights were 0.02226 g, 0.02487 g, 0.02620 g, and 0.00422 g for MgCl₂, *Pseudomonas PfBIO_Fn1, Pseudomonas PfBIO_Fn2* and *Pseudomonas* auxin-producer treated runners, respectively. This equates to 11.75%, 17.72 %, and -81.04 % differences in average rooting weights compared to application solution only for *PfBIO_Fn1, PfBIO_Fn2,* and the PfBIO auxin-producer strain, respectively. Rooting success was 83.33%, 90%, 90%, and 43.33%, respectively.

### Example 17 - Additional pathogen inhibition assay

In accordance with the methodology set out in Example 9, additional inhibition assays were conducted to evaluate the activity of *Pseudomonas PfBIO_Fn1* and *Pseudomonas PfBIO_Fn2* against various bacterial, fungal, and oomycete pathogens.

The results are summarised in the table below and Figure 15:
- active inhibition indicated by Y; and
- no inhibition indicated by N.

The table also specifies the crops from which each pathogen was isolated.

| **Pathogen** | ***PfBIO_Fn1*** | ***PfBIO_Fn2*** | **Crop of pathogen isolation** |
|---|---|---|---|
| *Phytophthora cactorum* | Y | Y | strawberry |
| *Phytophthora infestans* | Y | Y | potato |
| *Phytophthora capsici* | Y | Y | tomato |
| *Phytophthora plurivora* | Y | Y | lavender |
| *Botrytis cinerea* | Y | Y | strawberry |
| *Rhizoctonia solani* | Y | Y | potato |
| *Alternaria solani* | Y | Y | potato |
| *Fusarium oxysporum* | Y | Y | onion |
| *Fusarium solani* | Y | Y | potato |
| *Penicillium digitatum* | Y | Y | lemon |
| *Verticillium nonalfalfae* | Y | Y | hops |
| *Streptomyces scabies* | Y | Y | potato |
| *Xanthomonas campestris* | N | Y | cabbage |
| *Dickeya solani* | N | Y | potato |

## Claims

1. A method of treating or preventing a eukaryotic and/or bacterial infection in a plant or promoting growth in a plant, the method comprising administering a *Pseudomonas* bacterium, or extract thereof, to the plant.

2. The method of claim 1, wherein:
a) the eukaryotic infection is caused by a eukaryotic pathogen of the class *Oomycete,* optionally wherein the eukaryotic pathogen is of the genus *Phytophthora* and optionally wherein the eukaryotic pathogen is *Phytophthora cactorum, Phytophthora infestans, Phytophthora capsici* and/or *Phytophthora plurivora;* or
b) the eukaryotic infection is a fungal infection, optionally wherein the fungal infection is caused by a fungal pathogen of the genus *Rhizoctonia, Alternaria, Botrytis, Podosphaera, Fusarium, Penicillium* and/or *Verticillium,* optionally wherein the fungal pathogen is *Rhizoctonia solani, Alternaria solani, Botrytis cinerea, Podosphaera aphanis, Fusarium oxysporum, Fusarium solani, Penicillium digitatum* and/or *Verticillium nonalfalfae*; or
c) the bacterial infection is caused by a bacterial pathogen of the genus *Pectobacterium, Streptomyces, Xanthomonas* and/or *Dickeya,* optionally wherein the bacterial is *Streptomyces scabies, Xanthomonas campestris* and/or *Dickeya solani.*

3. The method of claim 1, wherein the promoted growth of the plant occurs in the leaves, stems, flowers, fruits and/or roots of the plant.

4. The method of any of the preceding claims, wherein the *Pseudomonas* bacterium, or extract thereof, is applied to the leaves, stems, flowers, fruits, tubers and/or roots of the plant.

5. The method of any of the preceding claims, wherein the *Pseudomonas* bacterium, or extract thereof, is applied to the substrate surrounding the plant to be administered to the plant, optionally wherein the substrate is soil, coir and/or an aqueous solution.

6. The method of any of the preceding claims, wherein the *Pseudomonas* bacterium has a 16S RNA gene with at least 80%, at least 85%, at least 90%, at least 95%, at least 98%, and/or at least 99% sequence identity with SEQ ID NOs 1 or 2.

7. The method of any of the preceding claims, wherein the *Pseudomonas* bacterium is *PfBIO_Fn1* (accession number 507455) and/or *PfBIO_Fn2* (accession number 507456).

8. The method of any of the preceding claims, wherein the *Pseudomonas* bacterium, or extract thereof, is administered to the plant at intervals of at least every 3 days, every 5 days, every 7 days, every 14 days, every 21 days, every 4 weeks, every month, every 2 months and/or every 3 months.

9. The method of any of the preceding claims, wherein the administration of the *Pseudomonas* bacterium, or extract thereof, does not adversely impact the health and/or growth of the plant.

10. A method of producing a *Pseudomonas* extract, the method comprising:
a) culturing a *Pseudomonas* bacterium under suitable conditions;
b) soaking the *Pseudomonas* culture in an extraction solution;
c) filtering the *Pseudomonas* culture and extraction solution;
d) drying the *Pseudomonas* filtrate; and
e) resuspending the *Pseudomonas* filtrate.

11. The method of claim 10, wherein:
(a) the *Pseudomonas* bacterium is *PfBIO_Fn1* (accession number 507455) and/or *PfBIO_Fn2* (accession number 507456); and/or
(b) the suitable conditions comprise incubating the *Pseudomonas* bacterium at 28°C; and/or
(c) the extraction solution is 50% v/v methanol in water, ethyl acetate; or 50% v/v acetonitrile in water; and/or
(d) the *Pseudomonas* culture and extraction solution are soaked for at least 2 hours, at least 6 hours, at least 12 hours or at least 24 hours; and/or
(e) the *Pseudomonas* culture and extraction solution are soaked at 4°C; and/or
(f) the *Pseudomonas* filtrate is resuspended in methanol, optionally wherein the *Pseudomonas* filtrate is resuspended in 50% v/v methanol in water.

12. An isolated *Pseudomonas* bacterium of *PfBIO_Fn1* (accession number 507455) and/or *PfBIO_Fn2* (accession number 507456).

13. Use of a *Pseudomonas* bacterium for treating or preventing a eukaryotic and/or bacterial infection in a plant and/or for promoting growth in a plant.

14. The use of claim 13, wherein the *Pseudomonas* bacterium:
a) has a 16S RNA gene with at least 80%, at least 85%, at least 90%, at least 95%, at least 98%, and/or at least 99% sequence identity with SEQ ID NOs 1 or 2; and/or
b) is *PfBIO_Fn1* (accession number 507455) and/or *PfBIO_Fn2* (accession number 507456).

15. A composition comprising an isolated *Pseudomonas* bacterium of claim 12 or a *Pseudomonas* extract obtained by the method of claim 10 or claim 11.
